# EUROPÄISCHE PATENTSCHRIFT

(11) **EP 2 540 237 B1**
(45) Veröffentlichungstag und Bekanntmachung des Hinweises auf die Patenterteilung: **21.01.2015**
(21) Anmeldenummer: 12004557.0
(22) Anmeldetag: 18.06.2012
(51) Int. Cl.: A61B 17/17

(54) **Vorrichtung zum Anzielen und Einbringen von Bohrkanälen in die Tibia**
Device for targeting and creating drilled channels in the tibia
Dispositif de vissage et d'introduction de canaux de forage dans le tibia

(30) Priorität: 28.06.2011 DE 102011106729
(43) Veröffentlichungstag der Anmeldung: 02.01.2013
(73) Patentinhaber: Karl Storz GmbH & Co. KG, 78532 Tuttlingen (DE)
(72) Erfinder: Berberich, Sascha, 78532 Tuttlingen (DE)
(74) Vertreter: Hofmeister, Frank

(56) Entgegenhaltungen:
- DE-A1-102007 057 075
- US-A- 5 968 050
- US-A1- 2003 216 742

## Beschreibung

Die Erfindung betrifft eine Vorrichtung zum Anzielen und Einbringen von Bohrkanälen in die Tibia im Bereich eines Kniegelenks bei der Rekonstruktion eines vorderen Kreuzbandes, mit einer Handhabe, die eine Bohrhülse zum Ausbilden eines ersten Zielpunktes an der Tibia sowie einen über das distale Ende der Bohrhülse vorstehenden Arm zum Bestimmen eines zweiten Zielpunktes am Tibiaplateau aufweist.

Eine gattungsgemäße tibiale Zielvorrichtung ist beispielsweise aus der DE 10 2007 057 075 A1 bekannt.

Das vordere Kreuzband, das sich vom oberen Plateau (Tibiaplateau) des Unterschenkelknochens (Tibia) bis zur Innenseite des unteren Endes des Oberschenkelknochens (Femur) erstreckt, bildet zusammen mit dem hinteren Kreuzband die beiden wichtigsten Bänder, die das Kniegelenk halten.

Aufgrund dieser wichtigen Haltefunktion des vorderen Kreuzbandes ist es bei einem Riss des vorderen Kreuzbandes erforderlich, das vordere Kreuzband durch eine natürliche Sehne oder durch ein Sehnenimplantat zu rekonstruieren. Hierzu wird von der Außenseite der Tibia her eine Bohrung in den Knochen eingebracht, die in Höhe des Tibiaplateaus an der Stelle austritt, an der das natürliche Kreuzband angewachsen ist.

Die Bohrung wird dann durch den Femur hindurchgeführt, bis diese an dessen Außenseite austritt. In die beiden Bohrungen wird anschließend das Sehnenimplantat bzw. das Ersatzband eingesetzt und so befestigt, dass dieses die Funktion des natürlichen vorderen Kreuzbandes übernehmen kann.

Für die erfolgreiche Rekonstruktion des vorderen Kreuzbandes ist entscheidend, dass der von der Außenseite der Tibia her zum Tibiaplateau erstellte Bohrkanal in einer anatomischen Ausrichtung steht, die der Ausrichtung des natürlichen Kreuzbandes bei einer bestimmten Kniestellung möglichst nahe kommt.

Da das Tibiaplateau aber für jeden Patienten anatomische Individualitäten aufweist, werden an die Ausbildung einer möglichst anatomischen Ausrichtung des Bohrkanals und somit auch an eine Zielvorrichtung zur Ausrichtung des Bohrkanals besondere Anforderungen gestellt.

Davon ausgehend liegt der Erfindung die **Aufgabe** zugrunde, eine Vorrichtung der eingangs genannten Art zu schaffen, die an die individuellen anatomischen Gegebenheiten des Patienten anpassbar ist.

Die **Lösung** dieser Aufgabenstellung ist erfindungsgemäß gekennzeichnet durch die Merkmale des Patentanspruchs 1. Hierbei ist mindestens ein Teilbereich des vertikalen Abschnitts des Arms reversibel elastisch verformbar ausgebildet.

Vorteilhafte Weiterbildungen der Erfindung sind Gegenstand der Unteransprüche.

Durch die elastische Verformbarkeit mindestens eines Teilbereichs des vertikalen Abschnitts des Arms ist es erstmalig möglich, den Auflagewinkel des distalen Endes des Arms zum Bestimmen des Zielpunktes am Tibiaplateau an die jeweilige individuelle anatomische Ausgestaltung des Tibiaplateaus des Patienten anzupassen. Um die Zielgenauigkeit des erfindungsgemäßen tibialen Zielgeräts aber trotz der individuellen Anpassbarkeit beizubehalten, ist der Arm der Handhabe nicht in Gänze, sondern nur teilbereichsweise verformbar ausgebildet.

Der Teilbereich des Arms ist reversibel verformbar ausgebildet, um das erfindungsgemäße tibiale Zielgerät für weitere individuelle Anpassungen verwenden zu können.

Der reversibel elastisch verformbare Teilbereich des Arms besteht aus einer Formgedächtnislegierung. Formgedächtnislegierungen oder auch Memorymetalle genannte Legierungen weisen die Eigenschaft auf, dass diese sich nach einer Verformung quasi an ihre Ausgangsform erinnern und aufgrund einer temperaturabhängigen Gitterumwandlung wieder in ihre Ausgangsform überführbar sind.

Diese temperaturbedingte Rückstellung der Verformung kann beispielsweise nach der Operation bei der thermischen Sterilisation des tibialen Zielgeräts erfolgen.

Schließlich wird mit der Erfindung vorgeschlagen, dass die Formgedächtnislegierung eine NiTi-Legierung, insbesondere Nitinol, ist.

Um sicherzustellen, dass es trotz der individuellen Anpassung des zweiten Zielpunktes an die Höhe des Tibiaplateaus zu keinem starken bzw. relevanten seitlichen Versatz kommt, ist gemäß der Erfindung weiterhin vorgesehen, dass der Arm im proximalen Endbereich einen im Wesentlichen parallel zur Handhabe verlaufenden senkrechten Abschnitt aufweist und, dass der mindestens eine verformbare Teilbereich des Arms in diesem vertikalen Abschnitt angeordnet ist.

Weitere Merkmale und Vorteile der Erfindung ergeben sich anhand der zugehörigen Zeichnung, in der ein Ausführungsbeispiel einer erfindungsgemäßen Vorrichtung zum Anzielen und Einbringen von Bohrkanälen in die Tibia nur beispielhaft dargestellt ist, ohne die Erfindung auf dieses Ausführungsbeispiel zu beschränken. In der Zeichnung zeigt:
- Fig. 1: eine perspektivische Ansicht einer erfindungsgemäßen Vorrichtung zum Anzielen und Einbringen von Bohrkanälen in die Tibia und
- Fig. 2: den Einsatz der Vorrichtung gemäß Fig. 1 beim Ausbilden eines ersten tibialen Bohrkanals.

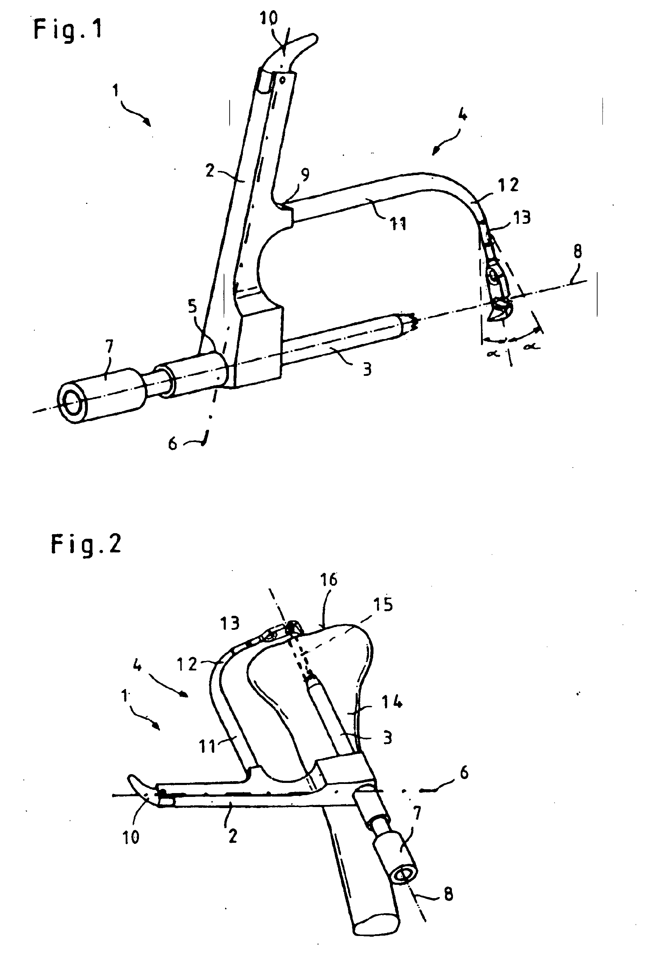

Die Abbildung Fig. 1 zeigt eine Vorrichtung 1 zum Anzielen und Einbringen von Bohrkanälen in die Tibia. Diese tibiale Zielvorrichtung 1 besteht aus einer stabförmigen Handhabe 2, an der zwei Zielhilfen lösbar angeordnet sind, nämlich eine Bohrhülse 3 und ein Arm 4.

Die Bohrhülse 3 ist an einem freien Ende der Handhabe 2 so angeordnet, dass sie in einer Bohrung 5 der Handhabe 2 gelagert, sich im Wesentlichen quer zur Längsachse 6 der Handhabe 2 erstreckt. Über eine auf der Bohrhülse 3 angeordneten Klemmhülse 7 ist die Klemmhülse in ihrer Lagerung in der Handhabe 2 so lösbar und wieder fixierbar, dass die Bohrhülse 3 in Richtung ihrer Längsachse 8 hin und her verschiebbar ist und auch vollständig aus der Bohrung 5 der Handhabe 2 entnehmbar ist.

Der die zweite Zielhilfe bildende Arm 4 zweigt bei der dargestellten Ausführungsform in etwa in halber Höhe von der stabförmigen Handhabe 2 ab. An der Handhabe 2 ist der Arm 4 in einer Aufnahme 9 gelagert, in der der Arm 4 über einen am der Bohrhülse 3 gegenüberliegenden freien Ende der Handhabe 2 angeordneten Klemmhebel 10 festlegbar ist.

Alternativ zu der dargestellten Ausbildung der Aufnahme 9 zur Lagerung des Arms 4 ist es selbstverständlich auch möglich, den Arm 4 über eine andere Halterung an der Handhabe 2 festzulegen oder sogar den Arm 4 einstückig mit der Handhabe 4 auszubilden.

Der Arm 4 weist einen im Wesentlichen parallel zur Bohrhülse 3 verlaufenden und an der Handhabe 2 gelagerten horizontalen Abschnitt 11 und einen sich distal daran anschließenden, sich in Richtung der Bohrhülse 3 erstreckenden vertikalen Abschnitt 12 auf, wobei das freie Ende des vertikalen Abschnitts 12 das distale Ende der Bohrhülse 3 in Richtung der Längsachse 8 überragt und in etwa in Höhe der Längsachse 8 angeordnet ist. Der vertikale Abschnitt 12 bildet den distalen Endbereich des Arms 4 und verläuft im Wesentlichen parallel zur Handhabe 2.

Wie weiterhin aus Fig. 1 ersichtlich, weist der vertikale Abschnitt 12 des Arms 4 einen Teilbereich 13 auf, der aus einer Formgedächtnislegierung gebildet reversibel elastisch verformbar ist. Mögliche Auslenkwinkel α nach distal und nach proximal sind in Fig. 1 gestrichelt dargestellt. Diese Anordnung des Teilbereichs 13 stellt sicher, dass die individuelle Anpassung im Wesentlichen nur auf die Höhe des Tibiaplateaus Einfluss nimmt, nicht aber zu einem starken bzw. relevanten seitlichen Versatz führt.

Alternativ zur Verwendung nur eines verformbaren Teilbereichs 13 ist es jedoch auch möglich, mehrere Teilbereiche 13 des Arms 4 elastisch verformbar auszubilden.

Formgedächtnislegierungen oder auch Memorymetalle genannte Legierungen weisen die Eigenschaft auf, dass diese sich nach einer Verformung quasi an ihre Ausgangsform erinnern und aufgrund einer temperaturabhängigen Gitterumwandlung wieder in ihre Ausgangsform überführbar sind.

Diese temperaturbedingte Rückstellung des verformten Teilbereichs 13 des Arms 4 kann beispielsweise nach der Operation bei der thermischen Sterilisation der Zielvorrichtung 1 erfolgen. Ein bevorzugtes Material zur Ausbildung einer derartigen Formgedächtnislegierung sind NiTi-Legierungen, insbesondere Nitinol.

Fig. 2 zeigt die Zielvorrichtung 1 in einer an die Tibia 14 (den Unterschenkelknochen) angelegten Stellung zur Ausbildung eines ersten Bohrkanals 15. Das distale Ende der Bohrhülse 3 markiert einen ersten Zielpunkt an der Außenseite der Tibia 14, der knapp unterhalb der Aufweitung der Tibia 14 liegt.

Das distale Ende des Arms 4 liegt am Tibiaplateau 16 und bildet den zweiten Zielpunkt der gleichzeitig den Austrittspunkt des Bohrkanals 15 bildet.

Da das Tibiaplateau 16 für jeden Patienten anatomische Individualitäten aufweist, ist es zur Ausbildung einer möglichst anatomischen Ausrichtung des Bohrkanals 15 vorteilhaft, dass mindestens ein Teilbereich 13 des Arms 4 elastisch verformbar ausgebildet ist, um den Auflagewinkel des distalen Endes des Arms 4 zum Bestimmen des Zielpunktes am Tibiaplateau 16 an die jeweilige individuelle anatomische Ausgestaltung des Tibiaplateaus 16 des Patienten anpassen zu können. Um die Zielgenauigkeit der tibialen Zielvorrichtung 1 aber trotz der individuellen Anpassbarkeit beizubehalten, ist der Arm 4 der Handhabe 2 nicht in Gänze, sondern nur teilbereichsweise verformbar ausgebildet.

Eine solchermaßen ausgebildete tibiale Zielvorrichtung zeichnet sich dadurch aus, dass sie bei einfacher Handhabung an die individuellen anatomischen Gegebenheiten des Patienten anpassbar ist.

### Bezugszeichenliste

- 1: Vorrichtung / Zielvorrichtung
- 2: Handhabe
- 3: Bohrhülse
- 4: Arm
- 5: Bohrung
- 6: Längsachse (Handhabe)
- 7: Klemmhülse
- 8: Längsachse (Bohrhülse)
- 9: Aufnahme
- 10: Klemmhebel
- 11: horizontaler Abschnitt
- 12: vertikaler Abschnitt
- 13: Teilbereich
- 14: Tibia
- 15: Bohrkanal
- 16: Tibiaplateau
- α: Auslenkwinkel

## Patentansprüche

1. Vorrichtung zum Anzielen und Einbringen von Bohrkanälen (15) in die Tibia (14) im Bereich eines Kniegelenks bei der Rekonstruktion eines vorderen Kreuzbandes, mit einer Handhabe (2), die eine Bohrhülse (3) zum Ausbilden eines ersten Zielpunktes an der Tibia (14) sowie einen über das distale Ende der Bohrhülse (3) vorstehenden Arm (4) zum Bestimmen eines zweiten Zielpunktes am Tibiaplateau (16) aufweist, wobei der Arm (4) im distalen Endbereich einen im Wesentlichen parallel zur Handhabe (2) verlaufenden senkrechten Abschnitt (12) aufweist, **dadurch gekennzeichnet, dass** mindestens ein Teilbereich (13) dieses vertikalen Abschnitts (12) des Arms (4) zur Anpassung an die individuelle anatomische Ausgestaltung des Tibiaplateaus (16) reversibel elastisch verformbar ausgebildet ist, wobei der mindestens eine verformbare Teilbereich (13) des vertikalen Abschnitts (12) des Arms (4) aus einer Formgedächtnislegierung besteht.

2. Vorrichtung nach Anspruch 1, **dadurch gekennzeichnet, dass** die Formgedächtnislegierung eine NiTi-Legierung, insbesondere Nitinol, ist.

## Claims

1. Device for sighting and driving drill holes (15) into the tibia (14) in the area of a knee joint in reconstruction of an anterior cruciate ligament, with a handle (2) which has a drill sleeve (3) for forming a first sighting point on the tibia (14) as well as an arm (4) protruding beyond the distal end of the drill sleeve (3), for determining a second sighting point on the tibial plateau (16), wherein the arm (4) in the distal end area has a vertical section (12) extending substantially parallel to the handle (2),
**characterized in**
**that** at least a partial area (13) of this vertical section (12) of the arm (4) is formed so that it is capable of reversible elastic deformation for adaptation to the individual anatomic configuration of the tibial plateau (16), wherein the at least one deformable partial area (13) of the vertical section (12) of the arm (4) consists of a shape memory alloy.

2. Device according to Claim 1, **characterized in that** the shape memory alloy is a NiTi alloy, in particular nitinol.

## Revendications

1. Dispositif pour assurer la visée et la réalisation de canaux de perçage (15) dans le tibia (14), dans la région de l'articulation du genou, lors de la reconstruction d'un ligament croisé antérieur, le dispositif comprenant une poignée de manoeuvre (2), qui présente un canon de perçage (3) pour former un premier point de visée ou point-cible sur le tibia (14), ainsi qu'un bras (4) dépassant au-delà de l'extrémité distale du canon de perçage (3) et destiné à définir un deuxième point de visée ou point-cible sur le plateau tibial (16), le bras (4) présentant, dans la zone d'extrémité distale, un tronçon (12) qui lui est perpendiculaire et s'étendant sensiblement de manière parallèle à la poignée de manoeuvre (2),
**caractérisé**
**en ce qu'**au moins une zone partielle (13) de ce tronçon vertical (12) du bras (4) est d'une conception permettant sa déformation élastique réversible, en vue d'une adaptation à la configuration anatomique individuelle du plateau tibial (16), ladite au moins une zone partielle déformable (13) du tronçon vertical (12) du bras (4), étant réalisée en un alliage à mémoire de forme.

2. Dispositif selon la revendication 1, **caractérisé en ce que** l'alliage à mémoire de forme est un alliage NiTi, notamment connu sous la dénomination "Nitinol".
